# FASCICULE DE BREVET EUROPEEN

(11) **EP 3 760 155 B1**
(45) Date de publication et mention de la délivrance du brevet: **01.09.2021**
(21) Numéro de dépôt: 20179650.5
(22) Date de dépôt: 12.06.2020
(51) Int. Cl.: A61B 50/30

(54) **DOUBLE EMBALLAGE POUR OBJET DESTINÉ À DEMEURER STÉRILE**
DOPPELVERPACKUNG FÜR GEGENSTAND, DER STERIL BLEIBEN SOLL
DOUBLE PACKAGING FOR OBJECT INTENDED TO REMAIN STERILE

(30) Priorité: 03.07.2019 FR 1907412
(43) Date de publication de la demande: 06.01.2021
(73) Titulaire: A. Raymond et Cie, 38000 Grenoble (FR)
(72) Inventeur: CLAVEL, Maxime, 38450 Vif (FR)
(74) Mandataire: IP Trust

(56) Documents cités:
- WO-A2-01/92117
- US-A1- 2018 178 963
- US-A9- 2019 175 275

## Description

### DOMAINE DE L'INVENTION

La présente invention concerne le domaine des emballages notamment adaptés pour des objets destinés à demeurer stériles jusqu'à utilisation ou implantation. L'invention concerne en particulier un double emballage pour un objet de type implant, par exemple dentaire ou orthopédique.

### ARRIERE PLAN TECHNOLOGIQUE DE L'INVENTION

On connaît de l'état de la technique des emballages se présentant sous la forme de double enveloppe externe et interne de type sachet ou double coque, contenant un objet, en particulier une pièce médicale stérilisée. Depuis son départ d'une zone non stérile (zone de stockage) jusqu'à son arrivée dans une zone stérile (par exemple un bloc opératoire), l'emballage est contaminé au niveau de son enveloppe extérieure, il est donc important d'éviter tout risque de contamination par contact direct ou indirect entre cette enveloppe extérieure et l'enveloppe interne ou même l'objet lui-même.

Pour cela, il est connu qu'un premier opérateur ouvre l'enveloppe extérieure, en zone stérile, et fasse tomber l'enveloppe intérieure sur un plan de travail ; un deuxième opérateur ouvre ensuite l'enveloppe intérieure pour en retirer l'objet stérilisé. L'inconvénient de cette approche est que l'enveloppe intérieure, et donc l'objet qu'elle contient, sont susceptibles de tomber au sol de manière accidentelle, et ainsi d'être impactés par le choc et contaminés par des corps étrangers présents sur le sol.

Selon une autre approche, le premier opérateur peut ouvrir l'enveloppe externe et la maintenir de sorte que le deuxième opérateur saisisse l'enveloppe interne, en prenant garde de ne pas toucher l'enveloppe externe. C'est l'approche proposée par exemple dans le document WO2012/172215.

Le document US2019/175275A9 décrit un double emballage comprenant un contenant externe et un contenant interne.

### OBJET DE L'INVENTION

La présente invention propose une solution alternative à celles de l'état de la technique. L'invention porte sur un double emballage pour un objet, par exemple un implant orthopédique ou dentaire, destiné à demeurer stérile jusqu'à utilisation, ledit double emballage permettant à la fois un maintien dans le contenant externe du contenant interne accueillant l'objet, et une extraction dudit contenant interne simple et respectant les exigences de stérilité.

### BREVE DESCRIPTION DE L'INVENTION

L'invention concerne un double emballage pour un objet préférentiellement destiné à demeurer stérile jusqu'à utilisation, comprenant :
- un contenant externe creux s'étendant globalement selon un axe longitudinal et comprenant une première extrémité fermée, une deuxième extrémité ouverte et des parois reliant la première et la deuxième extrémité, la deuxième extrémité étant destinée à être obturée par un système de fermeture,
- un contenant interne, disposé à l'intérieur du contenant externe, comprenant un corps creux et un bouchon, l'objet étant destiné à être accueilli dans ledit corps.

Le double emballage est remarquable en ce que :
- le contenant externe comporte, pour fermer sa première extrémité, une membrane susceptible de se déformer lorsque qu'une pression lui est appliquée,
- la membrane présente une face extérieure et une face intérieure et est configurée de sorte qu'une pression appliquée sur sa face extérieure, selon l'axe longitudinal, pousse sa face intérieure contre le contenant interne et provoque la sortie de ce dernier au niveau de la deuxième extrémité du contenant externe, en l'absence du système de fermeture.

Selon d'autres caractéristiques avantageuses et non limitatives de l'invention, prises seules ou selon toute combinaison techniquement réalisable :
- les parois du contenant externe définissent une forme tubulaire, dont l'axe central est l'axe longitudinal et présentant une section carrée, rectangulaire, polygonale, circulaire ou elliptique ;
- les parois du contenant externe comprennent au moins une première nervure sur une surface intérieure, ladite première nervure étant adaptée pour maintenir le contenant interne coincé dans le contenant externe, lorsqu'aucune pression n'est appliquée sur la membrane ;
- la (au moins une) première nervure est positionnée du côté de la première extrémité du contenant externe, voire à proximité de ladite première extrémité ;
- la membrane est susceptible de se déformer élastiquement lorsque qu'une pression lui est appliquée ;
- la face extérieure de la membrane présente une forme convexe, lorsqu'aucune pression n'est appliquée sur la membrane ;
- la membrane présente une forme générale hémisphérique ou de demi-ellipsoïde ;
- la face intérieure de la membrane comporte au moins une deuxième nervure, adaptée pour maintenir le contenant interne coincé dans le contenant externe, lorsqu'aucune pression n'est appliquée sur la membrane ;
- le bouchon du contenant interne est muni d'un porte-objet, destiné à maintenir fixement l'objet à l'intérieur du corps ;
- le porte-objet est formé en un matériau plastique souple ou rigide, et présente une forme générale tubulaire définissant un logement dans lequel est maintenu l'objet ;
- les parois du contenant externe et le corps du contenant interne sont formés dans un matériau plastique rigide, tel que l'acrylonitrile butadiène styrène (ABS), le polycarbonate (PC), un mélange d'acrylonitrile butadiène styrène et de polycarbonate (ABS-PC), les polyesters ou co-polyesters, ou le polypropylène (PP) ;
- le bouchon du contenant interne est formé dans un matériau plastique souple, choisi parmi les élastomères thermoplastiques (TPE), le polystyrène-b-polyéthylène-butylène-b-polystyrène (SEBS) et le silicone, ou dans un matériau plastique rigide tel que l'acrylonitrile butadiène styrène (ABS), le polycarbonate (PC), un mélange d'acrylonitrile butadiène styrène et de polycarbonate (ABS-PC), les polyesters ou co-polyesters, ou le polypropylène (PP) ;
- la membrane du contenant externe est formée dans un matériau choisi parmi les élastomères thermoplastiques (TPE), le polystyrène-b-polyéthylène-butylène-b-polystyrène (SEBS) et le silicone ;
- la membrane est fixée sur les parois du contenant externe par surmoulage ou par bi-injection ;
- le double emballage comprend le système de fermeture, ce dernier étant formé par un opercule fixé sur les parois du contenant externe, au niveau de la deuxième extrémité, par operculage, par thermo-soudage ou par collage ;
- l'opercule est étanche ou poreux.

### BREVE DESCRIPTION DES FIGURES

D'autres caractéristiques et avantages de l'invention ressortiront de la description détaillée de l'invention qui va suivre en référence aux figures annexées sur lesquelles :
- La figure 1 présente une vue en perspective d'un double emballage conforme à la présente invention ;
- Les figures 2a et 2b présentent respectivement une vue en perspective et une vue en coupe d'un double emballage, conforme à la présente invention ;
- Les figures 3a et 3b présentent respectivement une vue en perspective et une vue en coupe d'un double emballage conforme à la présente invention, lorsqu'une pression est appliquée sur la membrane du contenant externe, pour extraire le contenant interne accueillant l'objet ;
- La figure 4 présente une vue en perspective d'un double emballage conforme à la présente invention.

Les figures sont des représentations schématiques qui, dans un objectif de lisibilité, ne sont pas nécessairement à l'échelle.

### DESCRIPTION DETAILLEE DE L'INVENTION

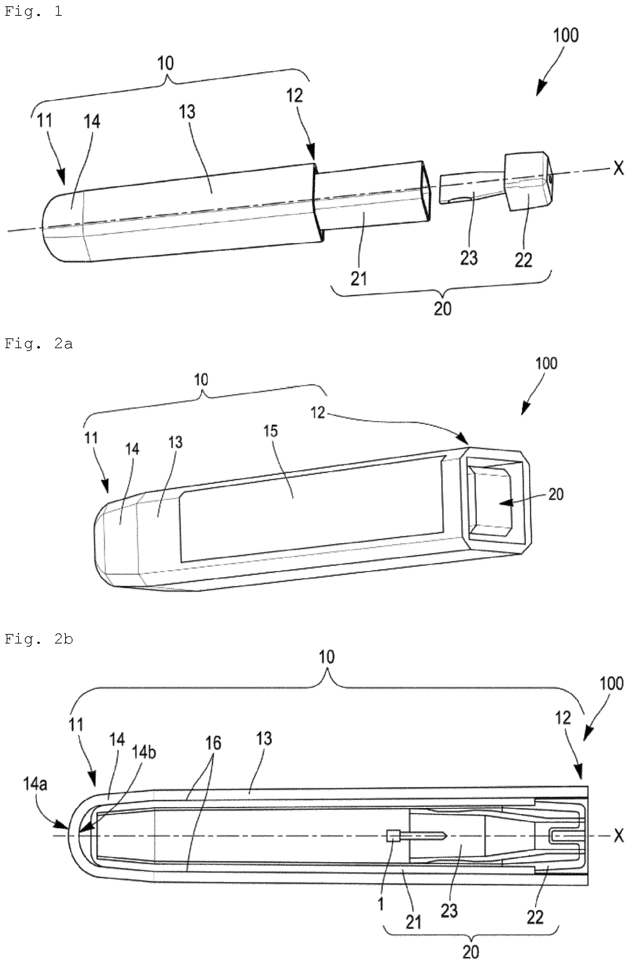

La présente invention concerne un double emballage 100 pour un objet 1 (visible sur la vue en coupe de la figure 2b) préférentiellement destiné à demeurer stérile jusqu'à utilisation. L'objet 1 pourra par exemple consister en une pièce médicale, un implant orthopédique ou dentaire.

Comme illustré sur la figure 1, le double emballage 100 comprend un contenant externe 10 creux s'étendant globalement selon un axe longitudinal X. Le contenant externe 10 comprend une première extrémité 11 fermée, une deuxième extrémité 12 ouverte et des parois 13 reliant la première 11 et la deuxième 12 extrémité. La deuxième extrémité 12 est destinée à être obturée de manière étanche par un système de fermeture (non représenté).

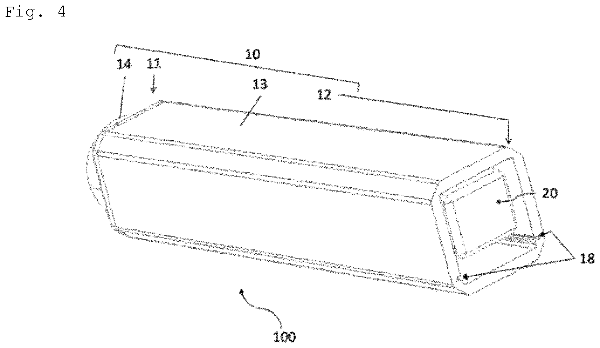

Les parois 13 du contenant externe 10 définissent une forme tubulaire, dont l'axe central est l'axe longitudinal X. La section du contenant externe 10 peut être de toute forme, par exemple carrée, rectangulaire, polygonale, circulaire ou elliptique. Dans les exemples illustrés, la section du contenant externe 10 est sensiblement carrée et présente des angles cassés au niveau de sa surface extérieure, comme cela est visible sur les figures 2a et 4. Ces angles cassés rendent le contenant externe 10 plus ergonomique et facilitent notamment la pose d'étiquette sur les parois 13 du contenant externe 10, en particulier au niveau de l'emplacement 15 (figure 2a).

Une alternative à la pose d'étiquettes sur une face extérieure des parois 13 du contenant externe 10 est l'aménagement d'encoches 18 à l'intérieur des parois 13, comme illustré en figure 4, adaptée pour loger une étiquette ou un petit livret. Ces encoches 18 forment une glissière dans laquelle l'étiquette ou le porte-étiquette est introduit. Cette alternative est avantageuse en ce qu'elle évite toute perte ou endommagement de l'étiquette lors du stockage du double-emballage 100 et conserve ladite étiquette en milieu stérile jusqu'à utilisation.

Pour fermer sa première extrémité 11, le contenant externe 10 comporte une membrane 14 susceptible de se déformer lorsque qu'une pression lui est appliquée. La membrane 14 est formée à partir d'un matériau souple et déformable. Avantageusement, elle est apte à subir une déformation élastique sous l'effet d'une pression et à retrouver sa forme initiale lorsque la pression n'est plus appliquée. La membrane 14 pourrait alternativement être formée dans un matériau susceptible de se déformer plastiquement lorsqu'une pression lui est appliquée, sans récupération de sa forme initiale, excluant ainsi toute réutilisation ultérieure.

La membrane 14 présente une face extérieure 14a et une face intérieure 14b. Nous reviendrons plus tard sur la forme de cette membrane 14 et sur les matériaux préférentiels dont elle peut être constituée.

Le double emballage 100 comprend également un contenant interne 20, lequel comprend un corps 21 creux et un bouchon 22, l'objet 1 étant destiné à être accueilli dans ledit corps 21. Préférentiellement, le bouchon 22 est monté en force au niveau de l'extrémité ouverte du corps 21. Alternativement, il pourrait être vissé, clippé ou se présenter sous forme d'un opercule scellé à l'extrémité du corps 21.

La forme et les dimensions du contenant interne 20 sont adaptées pour qu'il puisse être disposé à l'intérieur du contenant externe 10, sans frottement excessif contre la surface intérieure des parois 13.

Lorsqu'il est disposé dans le contenant externe 10, le contenant interne 20 y est complètement inclus et ne dépasse pas au niveau de la deuxième extrémité 12 (figures 2a et 2b). On comprend ainsi que le système de fermeture peut être placé au niveau de la deuxième extrémité 12, contre les parois 13 du contenant externe 10, pour obturer ce dernier et former un double emballage 100 clos et étanche, capable de contenir l'objet 1 stérile jusqu'au retrait du système de fermeture au moment de l'utilisation.

Selon un mode de réalisation avantageux, le système de fermeture est formé par un opercule fixé sur les parois 13 du contenant externe 10, au niveau de la deuxième extrémité 12. Différentes méthodes de fixation pourront être utilisées, dont notamment une méthode d'operculage, de thermo-soudage ou de collage.L'opercule peut être formé en différents matériaux, tels que par exemple à partir d'aluminium, de plastique, de complexes multi-matériaux ou encore de Tyvek™, textile synthétique non tissé composé de fibres de polyéthylène.

Selon un autre mode de réalisation, le système de fermeture est formé par un bouchon vissé, clippé ou monté en force.

Le (ou les) matériau (x) qui forme (nt) le système de fermeture pourra(ont) être choisi(s) étanche(s) ou poreux, selon le procédé de stérilisation visé.

En effet, lorsque l'objet 1 est disposé dans le contenant interne 20, lequel est inséré dans le contenant externe 10, et que le système de fermeture est en place sur les parois 13, le double emballage 100 peut subir une étape de stérilisation par exemple par rayons gamma. Les rayons gamma passent à travers les deux contenants 10,20 et tuent d'éventuelles bactéries, virus ou autres cellules vivantes présentes dans le contenant externe 10, dans le contenant interne 20, sur et éventuellement dans l'objet 1. Après cette étape de stérilisation, le double emballage 100 peut être stocké, jusqu'à son utilisation en salle d'opération stérile par exemple. Avec ce procédé de stérilisation, le système de fermeture est avantageusement choisi étanche. Alternativement, une stérilisation à l'oxyde d'éthylène (EtO) ou une stérilisation autoclave peut être appliquée au double emballage 100 muni du système de fermeture. Dans ce cas, le matériau dudit système est choisi poreux, de manière à permettre le passage des vapeurs de gaz ou d'eau, mais pas le passage de liquides ou autres contaminants. Le bouchon 22 du contenant interne 20 est lui aussi choisi poreux (par exemple sous forme d'opercule scellé au corps 21). A titre d'exemple, le système de fermeture du contenant externe 10 et le bouchon 22 du contenant interne 20 pourront dans ce cas se présenter sous forme d'opercules composées de Tyvek.

En référence à la figure 1, le bouchon 22 du contenant interne 20 est muni d'un porte-objet 23, destiné à maintenir fixement l'objet 1 à l'intérieur du corps 21. Le porte-objet 23 peut présenter différentes formes et comporter différents modes de fixation pour maintenir l'objet 1.

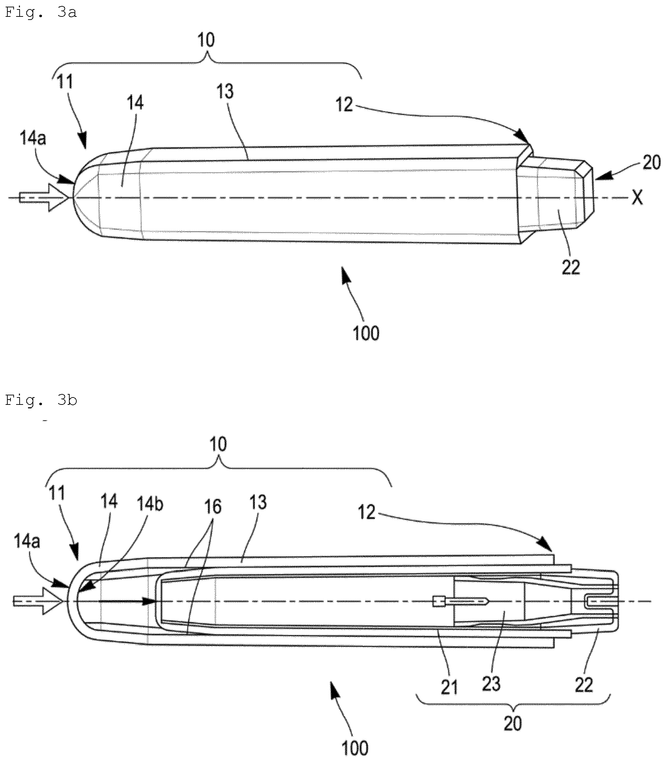

Selon un mode de réalisation avantageux, le porte-objet 23 est formé en un matériau souple et déformable, choisi par exemple parmi les élastomères thermoplastiques (TPE), le polystyrène-b-polyéthylène-butylène-b-polystyrène (SEBS) et le silicone. Il présente une forme générale tubulaire, définissant un logement de section ovale dans lequel est maintenu l'objet. Une extrémité du porte-objet 23 est maintenue solidaire du bouchon 22. L'autre extrémité du porte-objet 23 est ouverte et permet l'accès à l'objet 1. L'objet 1 pourra dépasser de l'extrémité ouverte du porte-objet 23 (comme illustré sur les figures 2b et 3b) ou alternativement être totalement inclus dans le logement formé par le porte-objet 23. Le matériau, l'épaisseur et les dimensions du porte-objet 23 sont choisis de sorte que les parois internes du porte-objet 23 soient plaquées contre l'objet 1 et l'empêchent, par frottement, de s'extirper de ce logement par gravité ou lorsque le double emballage 100 subit des secousses. A titre d'exemple, la section ovale du logement défini par le porte-objet 23 pourra présenter un petit diamètre inférieur ou égal à une dimension de l'objet 1, ce dernier se trouvant ainsi maintenu plaqué contre les parois internes du porte-objet 23. Selon un autre mode de réalisation, le porte-objet 23 est formé en un matériau rigide, par exemple plastique, tel que l'acrylonitrile butadiène styrène (ABS), le polycarbonate (PC), l'ABS-PC un mélange des composés précités, les polyesters ou co-polyesters, ou le polypropylène (PP). Il présente une forme générale tubulaire, définissant un logement dans lequel est maintenu l'objet 1. Une extrémité du porte-objet 23 est maintenue solidaire du bouchon 22. L'autre extrémité du porte-objet 23 est ouverte et autorise le maintien de l'objet 1 par exemple par vissage ; on peut accéder à l'objet 1 en le dévissant de cette extrémité.

Revenant à la description de la membrane 14, comme énoncé précédemment, ladite membrane 14 présente une face extérieure 14a et une face intérieure 14b. Elle est de plus configurée de sorte qu'une pression (schématisée par la flèche blanche, sur les figures 3a et 3b) appliquée sur sa face extérieure 14a, selon l'axe longitudinal X, pousse sa face intérieure 14b contre le contenant interne 20, et en particulier contre l'extrémité du corps 21. Cette poussée (schématisée par la flèche noire, sur la figure 3b) est possible du fait de la déformation (non représentée) de la membrane 14 dans une direction allant vers l'intérieur du contenant externe 10. En l'absence du système de fermeture, ladite poussée provoque la sortie du contenant interne 20, au niveau de la deuxième extrémité 12 du contenant externe 10.

Lorsque l'objet 1 doit être utilisé, le double emballage 100 est introduit dans la salle stérile par un premier opérateur, qui saisit la surface extérieure du contenant externe 10, laquelle surface est contaminée du fait de la station en zone de stockage du double emballage 100. En salle stérile, le premier opérateur retire le système de fermeture (par exemple, retrait d'un opercule scellé), ouvrant ainsi la deuxième extrémité 12 du contenant externe 10. Le premier opérateur applique ensuite une pression sur la face extérieure 14a de la membrane 14, selon l'axe longitudinal X : la face intérieure 14b de la membrane 14 entre alors en contact avec l'extrémité du corps 21 du contenant interne 20 et pousse ce dernier hors du contenant externe 10, via son extrémité 12 ouverte (figures 3a et 3b). Un deuxième opérateur (stérile celui-ci) peut alors saisir le contenant interne 20, totalement stérile, au niveau du bouchon 22 qui dépasse de la deuxième extrémité 12 du contenant externe 10.

Le double emballage 100 selon l'invention évite que le deuxième opérateur soit en contact avec la surface extérieure contaminée du contenant externe 10. Il facilite également la mise à disposition du contenant interne 20, par le premier opérateur (non stérile) au deuxième opérateur (stérile), sans choc ni risque de chute accidentelle et de contamination. Le contenant interne 20 est uniquement manipulé par le deuxième opérateur, en condition stérile, qui peut ensuite ouvrir le bouchon 22 et extraire l'objet 1 du porte-objet 23. L'extraction de l'objet 1 hors du porte-objet 23 est effectuée par simple pression sur les parois latérales du porte-objet 23, lorsque celui-ci est formé en matériau souple et déformable comme évoqué précédemment dans un mode de réalisation avantageux. En effet, une pression sur le porte-objet 23 est susceptible de le déformer et de l'élargir, relâchant ainsi l'objet 1.

Avantageusement, les parois 13 du contenant externe 10 comprennent au moins une première nervure 16 sur une surface intérieure (figures 2b et 3b) . La (au moins une) première nervure 16 est préférentiellement positionnée du côté de ou à proximité de la première extrémité 11 du contenant externe 10. Ladite première nervure 16 est adaptée pour maintenir le contenant interne 20 coincé dans le contenant externe 10, du fait du frottement mécanique entre la (ou les) première(s) nervure(s) 16 et la paroi du contenant interne 20, lorsqu'aucune pression n'est appliquée sur la membrane 14. La (au moins une) première nervure 16 pourra s'étendre parallèlement à l'axe longitudinal X ou selon un axe formant un angle non nul avec l'axe longitudinal X. Préférentiellement, plusieurs premières nervures sont uniformément réparties sur la surface intérieure des parois 13.

Du fait de la présence de cette (ou ces) première(s) nervure(s), il est indispensable d'appliquer une pression sur la membrane 14 pour pousser le contenant interne 20 et le décoincer afin de le faire sortir au niveau de la deuxième extrémité 12 du contenant externe 10. Ainsi, on évite les risques de sortie inopinée du contenant interne 20, hors du contenant externe 10, lorsque le système de fermeture a été retiré et lorsque la deuxième extrémité 12 ouverte du contenant externe 14 est, par inadvertance, orientée vers le bas.

La membrane 14 peut présenter différentes formes. Avantageusement, comme cela est illustré sur les figures annexées, la face extérieure 14a de la membrane 14 présente une forme convexe, dans son état « de repos », c'est-à-dire lorsqu'aucune pression ne lui est appliquée. Elle peut présenter une forme générale hémisphérique ou de demi-ellipsoïde. Préférentiellement, lorsque le contenant interne 20 est disposé intégralement dans le contenant externe 10 et que la membrane 14 est dans son état de repos, l'extrémité du corps 21 est en contact avec tout ou partie de la face intérieure 14b de ladite membrane 14, comme illustré en figure 2b.

Notons que la face intérieure 14b de la membrane 14 pourra comporter au moins une deuxième nervure, adaptée pour maintenir le contenant interne 20 coincé dans le contenant externe 10, par frottement entre ladite deuxième nervure et la paroi du contenant interne 20, lorsqu'aucune pression n'est appliquée sur la membrane 14 (c'est-à-dire dans son état de repos). La (au moins une) deuxième nervure pourra être mise en œuvre avec ou sans la (au moins une) première nervure 16.

La membrane 14 du contenant externe 10 pourra être formée dans un matériau choisi parmi les élastomères thermoplastiques (TPE), le polystyrène-b-polyéthylène-butylène-b-polystyrène (SEBS) et le silicone.

Les parois 13 du contenant externe 10 et le corps 21 du contenant interne 20 pourront être formés dans un matériau plastique rigide, tel que l'acrylonitrile butadiène styrène (ABS), le polycarbonate (PC), l'ABS-PC un mélange des composés précités, les polyesters ou co-polyesters, ou le polypropylène (PP).

Le bouchon 22 du contenant interne 20 pourra être formé dans un matériau souple choisi parmi les élastomères thermoplastiques (TPE), le polystyrène-b-polyéthylène-butylène-b-polystyrène (SEBS) et le silicone, ou dans un matériau rigide tel que l'acrylonitrile butadiène styrène (ABS), le polycarbonate (PC), l'ABS-PC un mélange des composés précités, les polyesters ou co-polyesters, ou le polypropylène (PP). Notons que si le bouchon 22 et le porte-objet 23 sont formés dans le même matériau, ils pourront être formés en une seule pièce plutôt qu'en deux pièces assemblées.

La membrane 14 est avantageusement fixée sur les parois 13 du contenant externe 10 par surmoulage ou par bi-injection, techniques bien connues pour la fabrication de pièces en matière(s) plastique(s).

Bien entendu, l'invention n'est pas limitée aux modes de mise en œuvre et exemples décrits, et on peut y apporter des variantes de réalisation sans sortir du cadre de l'invention tel que défini par les revendications.

## Revendications

1. Double emballage (100) pour un objet (1) préférentiellement destiné à demeurer stérile jusqu'à utilisation, comprenant :
- un contenant externe (10) creux s'étendant globalement selon un axe longitudinal (X) et comprenant une première extrémité (11) fermée, une deuxième extrémité (12) ouverte et des parois (13) reliant la première (11) et la deuxième (12) extrémité, la deuxième extrémité (12) étant destinée à être obturée par un système de fermeture,
- un contenant interne (20), disposé à l'intérieur du contenant externe (10), comprenant un corps creux (21) et un bouchon (22), l'objet (1) étant destiné à être accueilli dans ledit corps (21),
le double emballage (100) étant **caractérisé en ce que :**
- le contenant externe (10) comporte, pour fermer sa première extrémité (11), une membrane (14) susceptible de se déformer lorsque qu'une pression lui est appliquée,
- la membrane (14) présente une face extérieure (14a) et une face intérieure (14b) et est configurée de sorte qu'une pression appliquée sur sa face extérieure (14a), selon l'axe longitudinal (X), pousse sa face intérieure (14b) contre le contenant interne (20) et provoque la sortie de ce dernier au niveau de la deuxième extrémité (12) du contenant externe (10), en l'absence du système de fermeture.

2. Double emballage (100) selon la revendication précédente, dans lequel les parois (13) du contenant externe (10) définissent une forme tubulaire dont l'axe central est l'axe longitudinal (X) et présentant une section carrée, rectangulaire, polygonale, circulaire ou elliptique.

3. Double emballage (100) selon l'une des revendications précédentes, dans lequel les parois (13) du contenant externe (10) comprennent au moins une première nervure (16) sur une surface intérieure, ladite première nervure (16) étant adaptée pour maintenir le contenant interne (20) coincé dans le contenant externe (10), lorsqu'aucune pression n'est appliquée sur la membrane.

4. Double emballage (100) selon la revendication précédente, dans lequel la (au moins une) première nervure (16) est positionnée à proximité de la première extrémité (11) du contenant externe (10).

5. Double emballage (100) selon l'une des revendications précédentes, dans lequel la membrane (14) est susceptible de se déformer élastiquement lorsque qu'une pression lui est appliquée.

6. Double emballage (100) selon l'une des revendications précédentes, dans lequel la face extérieure (14a) de la membrane (14) présente une forme convexe, lorsqu'aucune pression n'est appliquée sur la membrane (14).

7. Double emballage (100) selon la revendication précédente, dans lequel la membrane (14) présente une forme générale hémisphérique ou de demi-ellipsoïde.

8. Double emballage (100) selon l'une des deux revendications précédentes, dans lequel la face intérieure (14b) de la membrane (14) comporte au moins une deuxième nervure, adaptée pour maintenir le contenant interne (20) coincé dans le contenant externe (10), lorsqu'aucune pression n'est appliquée sur la membrane (14).

9. Double emballage (100) selon l'une des revendications précédentes, dans lequel le bouchon (22) du contenant interne (20) est muni d'un porte-objet (23), destiné à maintenir fixement l'objet (1) à l'intérieur du corps (21).

10. Double emballage (100) selon la revendication précédente, dans lequel le porte-objet (23) est formé en un matériau plastique souple ou rigide, et présente une forme générale tubulaire définissant un logement dans lequel est maintenu l'objet (1).

11. Double emballage (100) selon l'une des revendications précédentes, dans lequel les parois (13) du contenant externe (10) et le corps (21) du contenant interne (20) sont formés dans un matériau plastique rigide, tel que l'acrylonitrile butadiène styrène (ABS), le polycarbonate (PC), un mélange d'acrylonitrile butadiène styrène et de polycarbonate (ABS-PC), les polyesters ou co-polyesters, ou le polypropylène (PP).

12. Double emballage (100) selon l'une des revendications précédentes, dans lequel le bouchon (22) du contenant interne (20) est formé dans un matériau plastique souple, choisi parmi les élastomères thermoplastiques (TPE), le polystyrène-b-polyéthylène-butylène-b-polystyrène (SEBS) et le silicone, ou dans un matériau plastique rigide tel que l'acrylonitrile butadiène styrène (ABS), le polycarbonate (PC), un mélange d'acrylonitrile butadiène styrène et de polycarbonate (ABS-PC), les polyesters ou co-polyesters, ou le polypropylène (PP).

13. Double emballage (100) selon l'une des revendications précédentes, dans lequel la membrane (14) du contenant externe (10) est formée dans un matériau choisi parmi les élastomères thermoplastiques (TPE), le polystyrène-b-polyéthylène-butylène-b-polystyrène (SEBS) et le silicone.

14. Double emballage (100) selon l'une des revendications précédentes, dans lequel la membrane (14) est fixée sur les parois (13) du contenant externe (10) par surmoulage ou par bi-injection.

15. Double emballage (100) selon l'une des revendications précédentes, comprenant le système de fermeture, ce dernier étant formé par un opercule fixé sur les parois (13) du contenant externe (10), au niveau de la deuxième extrémité (12), par operculage, par thermo-soudage ou par collage.

## Patentansprüche

1. Doppelverpackung (100) für ein Objekt (1), bevorzugt, bis zu einer Verwendung steril zu bleiben, umfassend:
- einen hohlen Außenbehälter (10), der sich im Allgemeinen gemäß einer Längsachse (X) erstreckt und ein erstes geschlossenes Ende (11), ein zweites offenes Ende (12) und Wände (13) umfasst, die das erste (11) und das zweite (12) Ende verbinden, wobei das zweite Ende (12) zum Verschliessen durch ein Schließsystem vorgesehen ist,
- einen Innenbehälter (20), der innerhalb des Außenbehälters (10) angeordnet ist, der einen hohlen Körper (21) und einen Verschluss (22) umfasst, wobei das Objekt (1) zur Aufnahme in dem Körper (21) vorgesehen ist,
wobei die Doppelverpackung (100) **dadurch gekennzeichnet ist, dass:**
- der Außenbehälter (10) zum Verschließen seines ersten Endes (11) eine Membran (14) aufweist, die zum Verformen, unter Druck, geeignet ist,
- die Membran (14) eine Außenfläche (14a) und eine Innenfläche (14b) aufrweist und so geeignet ist, dass ein auf ihre Außenfläche (14a) ausgeübter Druck gemäß der Längsachse (X) ihre Innenfläche (14b) gegen den Innenbehälter (20) schiebt und so bewirkt, dass dieser bei fehlendem Schließsystem hinsichtlich des zweiten Endes (12) des Außenbehälters (10) austritt.

2. Doppelverpackung (100) nach dem vorhergehenden Anspruch, wobei die Wände (13) des Außenbehälters (10) eine röhrenförmige Form definieren, deren Mittelachse die Längsachse (X) ist und einen quadratischen, rechteckigen, polygonalen, kreisförmigen oder elliptischen Abschnitt aufweist.

3. Doppelverpackung (100) nach einem der vorhergehenden Ansprüche, wobei die Wände (13) des Außenbehälters (10) wenigstens eine erste Rippe (16) an einer Innenfläche umfassen, wobei die erste Rippe (16) zum Beibehalten des Innenbehälters (20) geeignet ist, der in dem Außenbehälter (10) eingeklemmt ist, wenn kein Druck auf die Membran ausgeübt wird.

4. Doppelverpackung (100) nach dem vorhergehenden Anspruch, wobei die (wenigstens eine) erste Rippe (16) in der Nähe des ersten Endes (11) des Außenbehälters (10) positioniert ist.

5. Doppelverpackung (100) nach einem der vorhergehenden Ansprüche, wobei die Membran (14) zum elastischen Verformen, unter Druck, geeignet ist.

6. Doppelverpackung (100) nach einem der vorhergehenden Ansprüche, wobei die Außenseite (14a) der Membran (14) eine konvexe Form aufweist, wenn kein Druck auf die Membran (14) ausgeübt wird.

7. Doppelverpackung (100) nach dem vorhergehenden Anspruch, wobei die Membran (14) eine im Allgemeinen halbkugelförmige oder halbellipsoide Form aufweist.

8. Doppelverpackung (100) nach einem der zwei vorhergehenden Ansprüche, wobei die Außenseite (14b) der Membran (14) wenigstens eine zweite Rippe aufweist, die zum Beibehalten des Innenbehälters (20) geeignet ist, der in dem Außenbehälter (10) eingeklemmt ist, wenn kein Druck auf die Membran (14) ausgeübt wird.

9. Doppelverpackung (100) nach einem der vorhergehenden Ansprüche, wobei der Verschluss (22) des Innenbehälters (20) mit einem Objekthalter (23) versehen ist, der zum festen Beibehalten des Objekts (1) in dem Inneren des Körpers (21) vorgesehen ist.

10. Doppelverpackung (100) nach dem vorhergehenden Anspruch, bei der Objekthalter (23) aus einem flexiblen oder starren Kunststoffmaterial ausgebildet ist und eine allgemein röhrenförmige Form aufweist, die ein Gehäuse definiert, in dem das Objekt (1) beibehalten wird.

11. Doppelverpackung (100) nach einem der vorhergehenden Ansprüche, wobei die Wände (13) des Außenbehälters (10) und der Körper (21) des Innenbehälters (20) aus einem starren Kunststoffmaterial, wie zum Beispiel 1'Acrylnitril-Butadien-Styrol (ABS), Polycarbonat (PC), einer Mischung aus Acrylnitril-Butadien-Styrol und Polycarbonat (ABS-PC), Polyestern oder Co-Polyestern oder Polypropylen (PP) ausgebildet sind.

12. Doppelverpackung (100) nach einem der vorhergehenden Ansprüche, wobei der Verschluss (22) des Innenbehälters (20) aus einem flexiblen Kunststoffmaterial, das aus den thermoplastischen Elastomeren (TPE), Polystyrol-b-polyethylenbutylen-b-polystyrol (SEBS) und Silikon ausgewählt ist, oder aus einem starren Kunststoffmaterial, wie zum Beispiel Acrylnitril-Butadien-Styrol (ABS), Polycarbonat (PC), einer Mischung aus Acrylnitril-Butadien-Styrol und Polycarbonat (ABS-PC), Polyestern oder Co-Polyestern oder Polypropylen (PP) ausgebildet ist.

13. Doppelverpackung (100) nach einem der vorhergehenden Ansprüche, wobei die Membran (14) des Außenbehälters (10) aus einem Material ausgebildet ist, das aus den thermoplastischen Elastomeren (TPE), Polystyrol-b-polyethylenbutylen-b-polystyrol (SEBS) und Silikon ausgewählt ist.

14. Doppelverpackung (100) nach einem der vorhergehenden Ansprüche, wobei die Membran (14) an den Wänden (13) des Außenbehälters (10) durch Konfektionieren oder durch Bi-Injection befestigt ist.

15. Doppelverpackung (100) nach einem der vorhergehenden Ansprüche, die das Schließsystem umfasst, wobei dieses durch einen Deckel ausgebildet ist, der an den Wänden (13) des Außenbehälters (10) hinsichtlich des zweiten Endes (12) durch Konfektionieren, durch Heißsiegeln oder durch Kleben befestigt ist.

## Claims

1. Double packaging (100) for an object (1) preferably intended to remain sterile until use, comprising:
- a hollow external container (10) extending generally along a longitudinal axis (X) and comprising a first closed end (11), a second open end (12), and walls (13) connecting the first (11) and the second end (12), the second (12) end being intended to be sealed by a closure system,
- an internal container (20), placed inside the external container (10), comprising a hollow body (21) and a stopper (22), the object (1) being intended to be received in said body (21),
the double packaging (100) being **characterized in that:**
- to close its first end (11), the external container (10) includes a membrane (14) capable of deforming when pressure is applied to it,
- the membrane (14) has an outer face (14a) and an inner face (14b) and is configured so that a pressure applied to its outer face (14a), along the longitudinal axis (X), pushes its inner face (14b) against the internal container (20) and causes the latter to exit at the second end (12) of the external container (10), in the absence of the closure system.

2. Double packaging (100) according to the preceding claim, wherein the walls (13) of the external container (10) define a tubular shape of which the central axis is the longitudinal axis (X) and having a square, rectangular, polygonal, circular or elliptical section.

3. Double packaging (100) according to either of the preceding claims, wherein the walls (13) of the external container (10) comprise at least one first rib (16) on an interior surface, said first rib (16) being adapted to hold the internal container (20) wedged in the external container (10), when no pressure is applied to the membrane.

4. Double packaging (100) according to the preceding claim, wherein the (at least one) first rib (16) is positioned near the first end (11) of the external container (10).

5. Double packaging (100) according to one of the preceding claims, wherein the membrane (14) is capable of deforming elastically when pressure is applied to it.

6. Double packaging (100) according to one of the preceding claims, wherein the outer face (14a) of the membrane (14) has a convex shape, when no pressure is applied to the membrane (14).

7. Double packaging (100) according to the preceding claim, wherein the membrane (14) has a generally hemispherical or semi-elliptical shape.

8. Double packaging (100) according to one of the two preceding claims, wherein the internal face (14b) of the membrane (14) comprises at least one second rib, adapted to hold the internal container (20) wedged in the external container (10), when no pressure is applied to the membrane (14).

9. Double packaging (100) according to one of the preceding claims, wherein the stopper (22) of the internal container (20) is provided with an object holder (23), intended to fix the object (1) securely inside the body (21).

10. Double packaging (100) according to the preceding claim, wherein the object holder (23) is formed from a flexible or rigid plastics material, and has a generally tubular shape defining a housing in which the object (1) is held.

11. Double packaging (100) according to one of the preceding claims, wherein the walls (13) of the external container (10) and the body (21) of the internal container (20) are formed from a rigid plastics material, such as acrylonitrile butadiene styrene (ABS), polycarbonate (PC), a mixture of acrylonitrile butadiene styrene and polycarbonate (ABS-PC), polyesters or co-polyesters, or polypropylene (PP).

12. Double packaging (100) according to one of the preceding claims, wherein the stopper (22) of the internal container (20) is formed from a flexible plastics material, chosen from thermoplastic elastomers (TPE), polystyrene-b-polyethylene-butylene-b-polystyrene (SEBS), and silicone, or from a rigid plastics material such as acrylonitrile butadiene styrene (ABS), polycarbonate (PC), a mixture of acrylonitrile butadiene styrene and polycarbonate (ABS-PC), polyesters or co-polyesters, or polypropylene (PP).

13. Double packaging (100) according to one of the preceding claims, wherein the membrane (14) of the external container (10) is formed from a material chosen from thermoplastic elastomers (TPE), polystyrene-b-polyethylene-butylene-b-polystyrene (SEBS), and silicone.

14. Double packaging (100) according to one of the preceding claims, wherein the membrane (14) is fixed to the walls (13) of the external container (10) by overmolding or by bi-injection.

15. Double packaging (100) according to one of the preceding claims, comprising the closure system, the latter being formed by a seal fixed on the walls (13) of the external container (10), at the second end (12), by sealing, thermo-welding or bonding.
